# EUROPEAN PATENT APPLICATION

(11) **EP 4 039 258 A1**
(43) Date of publication of application: **10.08.2022**
(21) Application number: 21305168.3
(22) Date of filing: 08.02.2021
(51) Int. Cl.: A61K 31/5513, A61P 25/00, A61P 25/08, A61P 25/18, A61P 25/20, A61P 25/22, A61P 25/30

(54) **USE OF DIAZEPAM AS ONLY TOLERATED BENZODIAZEPINE RECEPTOR AGONIST IN COVID-19 PATIENTS**

(71) Applicant: ASSISTANCE PUBLIQUE, HOPITAUX DE PARIS, 75004 Paris (FR); Université de Paris, 75006 Paris 6 (FR)
(72) Inventor: Hoertel, Nicolas, 92130 Issy-les-Moulineaux (FR); Limosin, Frédéric, 75015 Paris (FR); Sanchez Rico, Marina Lucia, 92130 Issy-les-Moulineaux (FR)
(74) Representative: Regimbeau

(57) **Abstract**

The present invention is based on the finding that Benzodiazepine Receptor Agonists (BZRA), including benzodiazepines and Z-drugs, are significantly associated with increased mortality among patients hospitalized for COVID-19, with a significant dose-dependent relationship. It is therefore suggested to decrease the dose or to taper off these medications, when possible, in these patients. Importantly, this lethal association was not observed for only one BZRA, namely diazepam. This knowledge will help guide clinicians on the choice of the molecule to prescribe to patients with COVID-19 with clinical indication of a BZRA treatment (e.g. patients with benzodiazepine use disorder, delirium tremens, epilepsy, alcohol withdrawal symptoms, etc...).

## Description

### SUMMARY OF THE INVENTION

The present invention is based on the finding that Benzodiazepine Receptor Agonists (BZRA), including benzodiazepines and Z-drugs, are significantly associated with increased mortality among patients hospitalized for COVID-19, with a significant dose-dependent relationship. It is therefore suggested to decrease the dose or to taper off these medications, when possible, in these patients. Importantly, this lethal association was not observed for only one BZRA, namely diazepam. This knowledge will help guide clinicians on the choice of the molecule to prescribe to patients with COVID-19 with clinical indication of a BZRA treatment (e.g. patients with benzodiazepine use disorder, delirium tremens, epilepsy, alcohol withdrawal symptoms, etc...).

### BACKGROUND OF THE INVENTION

Global spread of the novel coronavirus SARS-CoV-2, the causative agent of coronavirus disease 2019 (COVID-19), has created an unprecedented infectious disease crisis worldwide 1-3

SARS-CoV-2 belongs to the species *Coronavirus*, in the genus *Betacoronavirus* and family *Coronaviridae.* Coronaviruses are enveloped viruses with a helically symmetrical capsid. They have a single-stranded, positive-sense RNA genome and are capable of infecting cells in birds and mammals. The morphology of the virions is typical, with a halo of protein protuberances ('Spike') which gave them their name of 'crown virus'. Among the four genera of coronaviruses, the Betacoronavirus genus (β-CoVs or Beta-CoVs), comprising virus infecting animals and/or humans, is subdivided into four lineages designated as A, B, C and D:
- Lineage A also designated as subgenus *Embecovirus* includes HCoV-OC43 and HCoV-HKU1, virus able to infect various species;
- Lineage B also designated as subgenus *Sarbecovirus* includes SARS-CoV-1, SARS-CoV-2, and Bat SL-CoV-WIV1;
- Lineage C also designated as subgenus *Merbecovirus* includes Tylonycteris bat coronavirus HKU4 (BtCoV-HKU4), Pipistrellus bat coronavirus HKU5 (BtCoV-HKU5), and MERS-CoV, able to infect notably camels and humans;
- Lineage D also designated as subgenus *Nobecovirus* includes Rousettus bat coronavirus HKU9 (BtCoV-HKU9).

The Betacoronaviruses of the greatest clinical importance concerning humans are:
- OC43 and HKU1 of the A lineage,
- SARS-CoV-1 and SARS-CoV-2 of the B lineage, and
- MERS-CoV of the C lineage.

In humans, coronavirus infections can cause respiratory pathologies associated with symptoms similar to the common cold, bronchiolitis and more serious diseases such as the Severe Acute Respiratory Syndrome caused by SARS-CoV-1, which generated an epidemic in 2003, and the Middle Eastern Respiratory Syndrome caused by MERS-CoV, which generated an epidemic in 2012. SARS-CoV-2 is the betacoronavirus causing the coronavirus epidemic of 2019-2021, generating the form of pneumonia known as coronavirus disease 2019 or COVID-19.

According to the World Health Organization (WHO), by January 25, 2021, 97 831 595 cases of COVID-19 have been confirmed and 2 120 877 patients have died worldwide. This epidemic was declared a public health emergency of international concern by WHO on January 30, 2020.

Symptoms of infection with SARS-CoV-2 are roughly similar to those of seasonal influenza infections: they include fever, fatigue, dry cough, shortness of breath, difficult breathing, pneumonia, renal failure, and may lead to death in severe cases.

The severity of clinical signs requires that approximately 20% of patients remain in hospital and 5% require admission to intensive care. However, this case-fatality rate remains uncertain, due to the difficulty in estimating the number of confirmed cases and deaths directly attributable to SARS-CoV-2 infection in the field. The most serious forms are observed in people who are vulnerable because of their age (over 70) or associated diseases such as hypertension, diabetes and/or coronary heart disease.

Benzodiazepine receptor agonists (BZRAs), including benzodiazepines and Z-drugs, potentiate the rapid neuroinhibitory effect of the neurotransmitter gamma-aminobutyric acid (GABA) in the brain and spinal cord. These medications are commonly prescribed for anxiety and sleep problems ⁴ and as muscle relaxants and pre-medications in anesthesia, and are also effective for treating epilepsy, alcohol withdrawal syndrome, and acute behavioral disturbance.⁵ Potential deleterious effects associated with these medications are well documented. Several studies, ⁶⁻¹² although not all, ¹³⁻¹⁶ have found a significant association between BZRAs and increased all-cause mortality. BZRAs have also been associated with increased risk of infection, ¹⁷ including pneumonia, ¹⁸ asthma exacerbation, ¹⁹ and respiratory depression. ²⁰

In this context, it was hypothesized that BZRA use would be associated with increased mortality among patients hospitalized for COVID-19, and that this association would be dose-dependent with higher doses conferring greater risk, and partially explained by respiratory depression. To test these hypotheses, the association between BZRAs and mortality was examined in the Assistance Publique-Hôpitaux de Paris (AP-HP) Health Data Warehouse, which collects data on all hospitalizations for COVID-19 in Greater Paris University hospitals since the beginning of the epidemic.

By so doing, the present inventors discovered that a number of BZRAs are indeed associated with increased mortality: of 14,381 adult patients hospitalized for COVID-19, 686 (4.8%) had received a BZRA within the first 48 hours of hospitalization at a mean daily diazepam-equivalent dose of 19.7 mg (SD=25.4). The primary endpoint was death. By comparing this endpoint with patients who did not received BZRAs, the analysis showed a significant association between BZRA use and increased mortality (HR=1.61; 95% CI=1.31-1.98, p<0.001), with a significant dose-effect relationship (HR=1.55; 95% CI=1.08-2.22; p=0.017). This association remained significant in multiple sensitivity analyses.

This association between BZRAs and mortality might be explained by several mechanisms. First, although the risk of respiratory impairment associated with benzodiazepines in the general population is debated, it might be relevantly high among elderly patients with COVID-19 and in those with pre-existing comorbidities, such as chronic obstructive pulmonary disease ⁴¹. Second, benzodiazepines may be associated with an increased risk of secondary infections, and particularly pneumonia, in patients with COVID-19 ⁴². Finally, in patients with COVID-19 and known risk factors for delirium (e.g., old age, dementia, multiple comorbidities), BZRA use may favor the occurrence of this condition⁴¹ which is associated with unfavorable COVID-19 disease prognosis ⁴³.

These first results suggested the need to carefully reevaluate the indication of each BZRA and decrease in dose or withdraw these medications when possible in patients with COVID-19, in line with professional guidelines recommendation for most patients on long term benzodiazepines ⁵.

However, BZRAs are known not only as efficient muscle relaxants and sedatives, but also effective for treating epilepsy, alcohol withdrawal syndrome, and acute behavioural disturbance ⁵. All these disorders are highly problematic in the handling of COVID patients, as COVID severe to most aggressive forms require a stay in intensive care unit (ICU) and therefore a quiet behavior for a long time. Alternative yet as efficient medications are therefore need to replace BZRAs in these applications.

In addition, in patients that are treated with BZRAs once they get infected, said treatment cannot be stopped brutally, as withdrawal side effects may occur, especially for patients already suffering from benzodiazepine use disorders or delirium tremens. In this context, substitutive treatments handling COVID 19 patients with clinical indication of BZRA prescription is crucial.

The association between mortality and BZRAs in COVID 19 patients, which was demonstrated for the first time by the inventors in the examples below, therefore highlighted the need to identify a drug that has the same therapeutic effects as BZRAs but that can be safely used in COVID19 patients or in subjects that are likely to be exposed to the SARS-COV-2 virus.

### DESCRIPTION OF THE INVENTION

Additional analyses performed by the inventors showed that, surprisingly, one BZRA treatment was not associated with an enhanced mortality in COVID 19 patients. As a matter of fact, when patients received diazepam prescribed at a mean daily diazepam-equivalent dose of 19.7 mg (SD=28.9, range=2.0 mg to 240.0 mg), mortality was found significantly lower than in patients receiving any other BZRA (HR=0.31; 95% CI=0.13-0.74; p=0.008) and not significantly different than in patients not receiving BZRAs (HR=0.84; 95% CI=0.29-2.46; p=0.747).

The finding that diazepam is not associated with increased mortality contrary to other BZRAs could be explained by several preclinical findings. A prior study suggests ⁴⁴ that the acid sphingomyelinase (ASM) / ceramide system may play a role in SARS-CoV-2 infections and that the formation of ceramide-enriched membrane platforms may mediate the entry of the virus into epithelial cells. Although most BZRAs do not influence cellular ceramide synthesis, diazepam has been shown to interact with the acyl coenzyme A binding protein, also known as 'diazepam binding inhibitor', a protein that contributes to ceramide synthesis ⁴⁵. This interaction may result in a reduced ceramide synthase and, finally, reduced cellular ceramide concentration.

Importantly, the importance of the acid sphingomyelinase (ASM) / ceramide system was also demonstrated for other receptor signaling and viral infection ⁴⁶;⁴⁷, including Rhinovirus ⁴⁸, Ebola virus ⁴⁹, measles ⁵⁰ and Japanese encephalitis virus ⁵¹, and non-viral infections such as *Pseudomonas aeruginosa* ⁴⁷*, Staphylococcus aureus* ⁵² and *Neisseriae gonorrhoeae* ^{53;54}. More precisely, Rhinovirus activates the ASM, induces ceramide and the formation of ceramide-enriched membrane domains, which serve as entrance for the virus. Both, amitriptyline and imipramine (functional inhibitors of ASM) block the infection of cells with rhinovirus ⁴⁸. A similar mechanism was reproduced for Ebola virus (imipramine, desipramine) ⁴⁹, measles (amitriptyline)⁵⁰ and Japanese encephalitis virus (amitriptyline, imipramine)⁵¹. Other viruses require the ASM/ceramide system for endosomal escape ⁵⁵. The ASM/ceramide mechanism also applies to non-viral infections such as *Pseudomonas aeruginosa* ⁴⁷*, Staphylococcus aureus* ⁵² and *Neisseriae gonorrhoeae* ^{53;54}.

Taking together, these results indicate that diazepam has to be preferred to other benzodiazepines among patients with clinical indication of a BZRA prescription, when said patient gets infected with several viruses or bacteria.

Therefore, the present inventors hereby propose to use Diazepam in patients suffering from a viral or bacterial infection, if and when they require any BZRA (i.e., either as a new treatment or as a switch instead of another BZRA, if this treatment is indicated). Said viral infection can be due for example to the SARS-COV-2 virus, to a Rhinovirus, the Ebola virus, the measles virus or the Japanese encephalitis virus. Said bacterial infection can be due to the bacteria *Pseudomonas aeruginosa, Staphylococcus aureus* or *Neisseriae gonorrhoeae.*

### Targeted subjects

In the context of the invention, the term "betacoronavirus" designates any virus belonging to the Betacoronavirus genus (β-CoVs or Beta-CoVs), in particular any betacoronavirus belonging to one of the four lineages designated as A, B, C and D. It designates a betacoronavirus infecting animals and/or humans. In particular, this designation includes the betacoronaviruses infecting human organisms chosen among OC43, HKU1, SARS-CoV-1, SARS-CoV-2 and MERS-CoV.

The term "viral infection due to at least one betacoronavirus" designates the fact that host cells of an organism have been infected by at least one betacoronavirus, the whole organism being said to have a viral infection.

A betacoronavirus infection in humans is usually diagnosed by a healthcare professional, based on observation of the infected patient's symptoms. Additional biological tests may be required to confirm the diagnosis: blood and/or sputum and/or bronchoalveolar fluid tests. Infection by a betacoronavirus can be established, for example, by molecular biological detection and/or viral titration of respiratory specimens, or by assaying blood for antibodies specific for said betacoronavirus. Conventional diagnostic methods comprise techniques of molecular biology such as PCR, which is well known to the person in the field.

In the context of the invention, the terms "COVID-19 disease" mean the disease linked to the infection with the SARS-CoV-2 (Severe Acute Respiratory Syndrome Coronavirus 2).

In the context of the invention, a "symptomatic COVID-19 disease" is characterized by a patient who shows at least one symptom of the COVID-19 disease. The most common symptoms of the COVID-19 disease are fever, muscle aches, headaches, fatigue, loss of taste and smell and respiratory symptoms such as a dry cough, difficulty breathing and a lack of oxygen. A symptomatic disease is in contrast to an asymptomatic disease which is characterized by a patient who is a carrier for a disease or infection but experiences no symptoms.

In particular, for the betacoronavirus disease (such as COVID-19), the following forms are usually observed:
- an **"asymptomatic form"** wherein the subject is a carrier of at least on betacoronavirus but shows no symptom.
- a **"mild COVID form"** wherein the subject shows the first symptoms (or signs) of a COVID disease, as listed above. Mild COVID symptoms (such as COVID-19) comprise e.g., mild dry cough, mild muscle pain, mild headache, mild fever, mild fatigue, loss of taste or smell.
- a **"strong COVID form"** wherein the subject shows strong respiratory symptoms such as difficulty breathing, lack of oxygen; other stronger symptoms such as fever, dry cough, aches and pains, nasal congestion, strong headache, conjunctivitis, sore throat, skin rash, discoloration of fingers or feet, or any combination thereof; as well as a deterioration of the general state of health with frequent diarrhoea, but also liver or urinary disorders, dizziness or neuromuscular problems; some of these symptoms may require hospitalisation. Most patients have an abnormal chest X-ray or CT scan within the first few days of illness, even in the absence of respiratory signs.
- A **"severe COVID form"** or **"critical COVID form"** or **"aggressive COVID form"** wherein the subject has life-threatening symptoms (or signs) of COVID (e.g. COVID-19), comprising at least one selected from (but not limited to): respiratory distress, lung disorders, liver disorders, kidney disorders, neuromuscular disorders, brain disorders, etc, that requires hospitalisation and/or intensive care (in intensive care units (ICU)).

In the context of the invention, the "severe symptomatic COVID-19 disease" is characterized by severe symptoms of the COVID-19 disease, in particular respiratory symptoms. Severe symptoms are acute respiratory distress syndrome that requires hospitalization of the patient in any unit, and especially in the intensive care unit (ICU) in case of critical infection. In the context of the invention, the abbreviation "ICU" refers to an intensive care unit, a special department of a hospital or health care facility that provides intensive treatment medicine.
In the context of the invention, "COVID-19 patients" are human patients infected with the SARS-CoV-2 (Severe Acute Respiratory Syndrome Coronavirus 2), whatever the form of the disease is. It means that it is possible to detect in their organism, and in particular in their blood and/or sputum and/or bronchoalveolar fluid, the presence of viral particles or genetic sequences, or of antibodies specific for said betacoronavirus. Conventional diagnostic methods comprise techniques of molecular biology such as PCR, which is well known to the person in the field. The said infection can be also diagnosed by viral titration of respiratory specimens, or by assaying blood for antibodies specific for said betacoronavirus.

These patients are also herein called "SARS-COV-2" positive subjects, because they have received a positive result with at least one of this conventional diagnostic method (e.g., PCR test or serologic test or an antigenic test).

In order to save time and avoid any prejudicial side effects upon or after BZRA administration, the methods of the invention can be also advantageously applied to any subject that is not yet infected by the SARS-COV-2 virus, but may have been exposed to said virus (close contact), or is likely to be exposed to the SARS-COV-2 virus in his / her daily routine (e.g., medical doctors, medical assistants, nurses, nursing assistants, home health aides, physicians, therapists, pharmacists, diagnostic medical sonographers, clinical laboratory technicians, dentists, dental assistants, emergency medical technicians, radiologic technologists, physical therapists, dental hygienists, respiratory therapists, anesthesiologists, obstetrician, surgeons, etc.). These subjects are herein called "SARS-COV-2 exposed".

In addition, "SARS-COV-2 exposed" subjects as meant in the present invention are subjects that have been in unprotected close contact with at least one SARS-COV-2 positive subject. By "unprotected close contact", it is herein meant that the subject has been exposed to the SARS-COV-2 virus, by being in contact with a SARS-COV-2 positive subject without carrying (or protected by) an effective protection at less than two meters, preferably less than one meter, whatever the duration of the contact was. Said effective protection can be a surgical mask, a FFP2 mask, a cloth mask or an hygiaphone or any other effective protection.

In such subjects, administering Diazepam instead of other BZRAs will prevent severe form of the COVID19 disease to develop, and possibly death, once the subject unfortunately gets infected with the SARS-COV-2 virus.

Alternatively, the methods and use of the invention may be applied to "SARS-COV-2 negative" subjects. The subjects should be "SARS-COV-2 negative" at least when the BZRA treatment (Diazepam or another BZRA) is administered. By "SARS-COV-2 negative", it is herein meant that the subject has received a negative result with at least one of the conventional diagnostic assay (e.g., PCR test or serologic test or an antigenic test), said assay being done no more than one day, preferably no more than few hours, before the BZRA treatment is administered. "SARS-COV-2 negative" subjects are more preferably individuals that have been efficiently immunized against the SARS-COV-2 virus, by natural immunization (earlier infection) or by vaccinal immunization. Even more preferably, they can also be inhabitants that live in a country where no SARS-COV-2 infection is expected (e.g., an island where SARS-COV-2 is not present).

In a preferred embodiment, these "SARS-COV-2 negative subjects" have been negatively tested for a SARS-COV-2 infection by molecular biological detection and/or viral titration of respiratory specimens, or by assaying blood for antibodies specific for said betacoronavirus.

In a preferred embodiment, said subject is a human patient. Said patient can be older than 75 years old, between 65 and 74 years old, between 18 and 65 years old, or below 18 years old. In a more preferred embodiment, said human patient is older than 50 years old, even more preferably older than 70 years old. These people indeed usually have greater mortality rates than younger people when infected with said virus.

### Administration of BZRA only in SARS-COV-2 negative subjects

As explained in the experimental part below, the present inventors discovered that COVID19 patients treated with BZRAs (except Diazepam) have an increased mortality. It is therefore not recommended, if not prohibited, to administer a BZRA (except Diazepam) to SARS-COV-2 positive subjects. Yet, it remains possible and beneficial to administer BZRA (including Diazepam) to SARS-COV-2 negative subjects.

In the present disclosure, the term "BZRA" encompasses drugs having a benzodiazepine chemical structure, as well as z-drugs.

In the context of the present invention, the "BZRA" (also called "BZRA drugs" or "BZRA compounds" or "BZRA agents") can be for example chosen in the group consisting of:
- Benzodiazepine molecules: alprazolam, bromazepam, chlordiazepoxide, cobazam, clonazepam, clorazepate, diazepam, flurazepam, lorazepam, midazolam, nitrazepam, oxazepam, temazepam, triazolam,
- Z-drugs: Imidazopyridines (Alpidem, Necopidem, Saripidem, Zolpidem), Pyrazolopyrimidines (Divaplon, Fasiplon, Indiplon, Lorediplon, Ocinaplon, Panadiplon, Taniplon, Zaleplon), Cyclopyrrolones (Eszopiclone, Pagoclone, Pazinaclone, Suproclone, Suriclone, Zopiclone), β-Carbolines (Abecarnil, Gedocarnil
   SL-651,498, or ZK-93423), and others (CGS-20625, CGS-9896, CL-218,872, ELB-139, GBLD-345, HIE-124, L-838,417, NS-2664, NS-2710, Pipequaline, RWJ-51204, SB-205,384, SL-651,498, SX-3228, TP-003, TP-13, TPA-023, Y-23684).

The recommendations made in the present invention deal with all these BZRAs (except Diazepam), as they have similar benefits, side effects, and risks.

These medications are commonly prescribed for anxiety and sleep problems and as muscle relaxants and pre-medications in anesthesia. They are also effective for treating epilepsy, alcohol withdrawal syndrome, and acute behavioral disturbance.

These drugs have regulatory approval for the management of anxiety and panic disorders, and short-term treatment of insomnia, seizures, benzodiazepine withdrawal, opiate withdrawal, alcohol withdrawal, sedation, and spasticity. They are also often used in an off-label manner to treat conditions such as anxious depression or the behavioral and psychological symptoms of dementia, which are also referred to as responsive behavior.

Thus, in this aspect of the invention, the subject to be treated may suffer from anxiety, panic disorders, insomnia, seizures, sedation, depression, acute behavioral disturbance, delirium, dementia and/or spasticity. Also, he/she may suffer from alcohol withdrawal, benzodiazepine withdrawal, or opiate withdrawal.

In one aspect, the present invention is thus drawn to any of these BZRA for use in the prevention and/or treatment of anxiety, panic disorders, insomnia, seizures, benzodiazepine withdrawal, opiate withdrawal, alcohol withdrawal, sedation, depression, acute behavioral disturbance, dementia and/or spasticity, wherein said BZRA is used only in SARS-COV-2 negative subjects. In other terms, the present invention relates to the use of any BZRA (including Diazepam), to prepare a medicament intended to treat patients suffering from anxiety, panic disorders, insomnia, seizures, alcohol withdrawal, benzodiazepine withdrawal, opiate withdrawal, sedation, depression, acute behavioral disturbance, delirium, dementia and/or spasticity, as long as they are not infected with the virus SARS-COV-2.

The present invention also discloses a method for preventing and/or treating anxiety, panic disorders, insomnia, seizures, alcohol withdrawal, benzodiazepine withdrawal, opiate withdrawal, sedation, depression, acute behavioral disturbance, delirium, dementia and/or spasticity in a subject, said method comprising administering to said subject an effective amount of any BZRA, only if he/she has not been diagnosed to be infected with the virus SARS-COV-2. The method of the invention can comprise a diagnosis step as preliminary step.

In this case, said method may comprise the following steps:
a) diagnosing if the subject is infected with the virus SARS-COV-2,
b) administering a BZRA only if the diagnostic step reveals that the subject is SARS-COV-2-negative (i.e., not infected by the SARS-COV-2 virus).

In these methods and uses, the treated subjects are preferably "SARS-COV-2 negative" when said BZRA treatment is administered. This means that, for example, they have received a negative result with at least one of the conventional diagnostic method (e.g., PCR test or serologic test or an antigenic test) few hours before the BZRA treatment is administered, or they have been efficiently immunized against the SARS-COV-2 virus by a COVID vaccine or by an earlier infection, or they live in a country where no SARS-COV-2 infection is present or expected (e.g., an island where said virus has not been detected or has been eradicated).

Especially, administering BZRAs remains recommended for treating anxiety, panic disorders, insomnia, seizures, alcohol withdrawal, benzodiazepine withdrawal, opiate withdrawal, sedation, depression, acute behavioral disturbance, delirium, dementia and/or spasticity in SARS-COV-2 immunized subject.

Also, because of the similar molecular pathways involved in the entry of these pathogens in human cells (involving the ASM/ceramide system), the present inventors hereby recommend not to use BZRAs in patients suffering from a viral or bacterial infection, said infection being due to a Rhinovirus, the Ebola virus, the measles virus or the Japanese encephalitis virus or to the bacteria *Pseudomonas aeruginosa, Staphylococcus aureus* or *Neisseriae gonorrhoeae.*

All the above detailed embodiments can also be applied to any of these viral or bacterial - infected subjects, to prevent strong infections and lethality to occur.

### Deprescribing BZRA for preventing severe forms of COVID19 to occur

In another aspect, the present invention is drawn to an *in vitro* method for preventing severe forms of COVID 19 disease to occur in a patient treated by a BZRA drug, said method comprising deprescribing said BZRA drug in said patient.

The present invention more precisely concerns a method for preventing severe forms of COVID19 disease to occur in a patient treated by a BZRA drug, said method comprising the steps of :
a) diagnosing if the subject is infected with the virus SARS-COV-2,
b) deprescribing BZRA if the diagnostic step reveals that the subject is SARS-COV-2-positive (i.e., infected by the SARS-COV-2 virus).

More precisely, step b) is to be applied as soon as said subject receives a positive result with at least one of this conventional diagnostic method (e.g., PCR test or serologic test or an antigenic test).

By "deprescribing" a BZRA drug, it is herein meant that the physician has one of the following options:
- Abruptly stopping the BZRA (i.e" abrupt discontinuation),
- Tapering the BZRA dose (i.e., gradually reducing the dose until complete cessation of the BZRA),
- Prescribing Diazepam instead (with the aim of stopping or reducing BZRA use in the process)
- Combining tapering and Diazepam, by cross-tapering.

Of course, if the patient was already treated with Diazepam, then there is no need to change the treatment. In some cases, it may be recommended to decrease the Diazepam dose, if possible, because this treatment (as well as all BZRAs) are only indicated for short-term use.

As explained above, administering diazepam instead of other BZRAs in subjects that are under treatment with BZRAs will help impairing anxiety, seizures, alcohol withdrawal syndrome, benzodiazepine withdrawal syndrome, muscle spasms, trouble sleeping, acute behavioral disturbance, dementia, delirium, restless legs syndrome, epilepsy, or will help inducing sedation before an invasive chirurgical operation in said subjects, and will prevent severe form of the COVID19 disease to develop, and possibly death to occur, if and once the subject gets infected with the SARS COV-2 virus. For these subjects, a better prognosis could be acknowledged, as compared to COVID patients still receiving their current dose of BZRA.

In a preferred embodiment, the present invention concerns a method for treating agitation, anxiety, panic, muscle spasms, benzodiazepine withdrawal, opiate withdrawal, alcohol withdrawal, trouble sleeping, dementia, delirium, acute behavioral disturbance, restless legs syndrome, or for preventing epileptic seizure and/or treating epilepsy, or for inducing pre-or post-operative sedation, anxiolysis, or amnesia in a subject in need thereof, said method comprising the steps of:
a) performing a PCR test or a serologic test or an antigenic test to detect if the subject is infected with the virus SARS-COV-2,
b) in case the subject is indeed positive for SARS-COV-2, administering Diazepam to said subj ect,
c) in the contrary case (i.e., the subject is not infected by the SARS-COV-2 virus), administering a BZRA drug to said subject.

Step b) or c) is to be applied as soon as said subject receives a positive result with at least one of this conventional diagnostic method (e.g., PCR test or serologic test or an antigenic test).

Step b) or c) is to be applied as soon as said subject receives a positive result with at least one of this conventional diagnostic method (e.g., PCR test or serologic test or an antigenic test).

In a preferred embodiment, the present invention concerns a method for treating agitation, anxiety, panic, muscle spasms, benzodiazepine withdrawal, opiate withdrawal, alcohol withdrawal, trouble sleeping, acute behavioral disturbance, dementia, delirium, restless legs syndrome or for preventing epileptic seizure and/or treating epilepsy, or for inducing pre-or post-operative sedation, anxiolysis, or amnesia in a subject in need thereof in a subject in need thereof, said method comprising the steps of :
a) determining if the subject has been exposed to the virus SARS-COV-2, e.g., by determining if the subject has been in unprotected close contact with a SARS-COV-2 positive subject, in the last two weeks,
b) in case the subject has been indeed exposed to said virus, administering Diazepam to said subj ect,
c) in the contrary case (i.e., the subject has not been exposed to the SARS-COV-2 virus in the last two weeks), administering a BZRA drug to said subject.

As explained above, BZRA drugs act as allosteric modulators of gamma-aminobutyric acid (GABA) activity by binding to inotropic benzodiazepine receptors at the GABA A receptor complex. They increase GABA binding and chloride ion channel opening, facilitating their inhibitory activity.

Some of these drugs have a benzodiazepine chemical structure (e.g., alprazolam, bromazepam, chlordiazepoxide, cobazam, clonazepam, clorazepate, diazepam, flurazepam, lorazepam, midazolam, nitrazepam, oxazepam, temazepam, triazolam), while others do not and are referred to as "non-benzodiazepine receptor agonists", "novel benzodiazepine receptor agonists", or "z-drugs" (e.g., zolpidem, zopiclone).

In the present disclosure, the term "BZRA" encompass drugs having a benzodiazepine chemical structure as well as z-drugs.

In the context of the present invention, the "BZRA drugs" are therefore chosen in the group consisting of:
- Benzodiazepine molecules: alprazolam, bromazepam, chlordiazepoxide, cobazam, clonazepam, clorazepate, diazepam, flurazepam, lorazepam, midazolam, nitrazepam, oxazepam, temazepam, triazolam,
- Z-drugs: Imidazopyridines (Alpidem, Necopidem, Saripidem, Zolpidem), Pyrazolopyrimidines (Divaplon, Fasiplon, Indiplon, Lorediplon, Ocinaplon, Panadiplon, Taniplon, Zaleplon), Cyclopyrrolones (Eszopiclone, Pagoclone, Pazinaclone, Suproclone, Suriclone, Zopiclone), β-Carbolines (Abecarnil, Gedocarnil, SL-651,498, or ZK-93423), and others (CGS-20625, CGS-9896, CL-218,872, ELB-139, GBLD-345, HIE-124, L-838,417, NS-2664, NS-2710, Pipequaline, RWJ-51204, SB-205,384, SL-651,498, SX-3228, TP-003, TP-13, TPA-023, Y-23684).

The recommendations made in the present invention deal with all these BZRAs, as they have similar benefits, side effects, and risks.

In this aspect of the invention, the patient may suffer from anxiety, panic disorders, insomnia, seizures, alcohol withdrawal, sedation, depression, acute behavioral disturbance, dementia, delirium, and/or spasticity, for which a BZRA was initially prescribed by a physician. Also, he/she may suffer from alcohol withdrawal, benzodiazepine withdrawal, or opiate withdrawal for which a BZRA was initially prescribed by a physician.

Also, because of the similar molecular pathways involved in the entry of these pathogens in human cells (involving the ASM/ceramide system), the present inventors hereby recommend to deprescribe BZRAs in patients suffering from a viral or bacterial infection, said infection being due to a Rhinovirus, the Ebola virus, the measles virus or the Japanese encephalitis virus or to the bacteria *Pseudomonas aeruginosa, Staphylococcus aureus* or *Neisseriae gonorrhoeae.*

All the above detailed embodiments can also be applied to any of these viral or bacterial - infected subjects, to prevent strong infections and lethality to occur.

### Therapeutic applications of Diazepam in agitated or epileptic COVID19 patients

The results obtained by the inventors showed that, surprisingly, mortality of patients receiving Diazepam at a mean daily diazepam-equivalent dose of 19.7 mg (SD=28.9, range=2.0 mg to 240.0 mg) was significantly lower than in patients receiving other BZRA. Thus, the only one BZRA not leading to an enhanced mortality in COVID 19 patients is Diazepam.

These results indicate that Diazepam has to be preferred to other benzodiazepines among patients with COVID-19 requiring this treatment.

In another aspect, the present invention relates to the Diazepam treatment of patients that have been infected by - or are exposed to - the SARS-COV-2 virus and experience (or may experience) agitation, anxiety, panic, muscle spasms, trouble sleeping, and restless legs syndrome. Preferably, these patients were not previously treated with any BZRA.

Importantly, this treatment can also benefit to patients that are at risk of delirium (due to old age or dementia or to multiple comorbidities), or those who are epileptic. In the latter case, administration of Diazepam, which is an anticonvulsant, will indeed prevent epileptic seizure to occur, for example if and once the patient is admitted in the Intensive Care Unit.

As shown in the examples below, this treatment should solely involve Diazepam and no other BZRA.

In this aspect, the present invention relates to Diazepam for use in preventing severe forms of COVID19 disease to occur in SARS-COV-2 positive subjects or SARS-COV-2 exposed subjects that are treated with a Benzodiazepine Receptor Agonist (BZRA) other than Diazepam.

Preferably, in this aspect, Diazepam is administered for its authorized or classical therapeutic indications, namely:
- preventing and/or treating agitation, anxiety, panic, muscle spasms, trouble sleeping, delirium, acute behavioral disturbance, dementia, and restless legs syndrome, or for
- preventing and/or treating benzodiazepine withdrawal, opiate withdrawal, alcohol withdrawal, or for,
- preventing epileptic seizure in and/or treating epileptic patients, or for
- inducing pre-or post-operative sedation, anxiolysis, or amnesia.

In other terms, the present invention relates to the use of Diazepam, to prepare a medicament intended to prevent severe forms of the COVID19 disease to occur in patients that have been diagnosed to be infected with the virus SARS-COV-2 (SARS-COV-2 positive subjects) or that have been exposed to the virus SARS-COV-2, should they suffer from agitation, anxiety, panic, muscle spasms, trouble sleeping, delirium, benzodiazepine withdrawal, opiate withdrawal, alcohol withdrawal, acute behavioral disturbance, dementia and restless legs syndrome, or from epilepsy, or should they have to undergo an operation.

The present invention also relates to Diazepam, for use in preventing and/or treating agitation, anxiety, panic, muscle spasms, trouble sleeping, delirium, benzodiazepine withdrawal, opiate withdrawal, alcohol withdrawal, acute behavioral disturbance, dementia, and restless legs syndrome in patients suffering from COVID19 (SARS-COV-2 positive subjects) or likely to suffer from the COVID19 (i.e., in SARS-COV-2 exposed subjects).

The present invention also relates to Diazepam, for use in preventing epileptic seizure and/or treating epileptic patients suffering from COVID19 (SARS-COV-2 positive subjects) or likely to suffer from the COVID19 (i.e., in SARS-COV-2 exposed subjects).

Diazepam is a well-known medicine marketed under several names: Valium^{®}, Valrelease^{®}, Vazepam^{®}, Diaz Intensol^{®}, Diastat^{®}, Dizac^{®}. Its chemical structure is 7-chloro-1,3-dihydro-1-methyl-5-phenyl-2H-1,4-benzodiazepin-2-one. As all 1,4-benzodiazepine, it binds with high affinity to the GABA A receptor in the brain to reduce arousal and to affect emotions. Diazepam's action causes an increase in affinity of the major inhibitory neurotransmitter, GABA. GABA binds mainly to the A subunit while Diazepam binds to the B subunit. Diazepam increases chloride transport through ion-channels and ultimately reduces the arousal of the cortical and limbic systems in the CNS. Diazepam depresses the electrical after-discharge in the amygdala and hippocampus regions of the limbic system that affect emotions.

In this aspect, Diazepam is preferably administered by systemic oral administration. Intravenous or intramuscular injection is also possible after preparing a solution from crushed tablets. Commercially available liquid Valium^{®} can be injected, and gel forms can be rectally administered.

In this aspect of the invention, Diazepam is intended to treat patients suffering from an asymptomatic or symptomatic COVID-19 disease, and advantageously from a strong or severe symptomatic COVID-19 disease, as defined above. In a particular embodiment, Diazepam is administered to SARS-COV-2 positive subjects that experience for example difficulty in breathing and/or a lack of oxygen, to prevent agitation, anxiety, panic, muscle spasms, trouble sleeping, epileptic seizure, benzodiazepine withdrawal, opiate withdrawal, alcohol withdrawal acute behavioral disturbance, dementia, delirium, and restless legs syndrome in same.

In SARS-COV-2 positive subjects, Diazepam will prevent a severe form of the COVID19 disease to develop, and will lower the risk of unvoluntary seizures or agitated states that may complicate the intubation protocols and therefore facilitate the treatment of the patients so as, eventually, to increase their survival rate.

Also, because of the similar molecular pathways involved in the entry of these pathogens in human cells (involving the ASM/ceramide system), the present inventors hereby recommend to use Diazepam to prevent agitation, anxiety, panic, muscle spasms, trouble sleeping, epileptic seizure, benzodiazepine withdrawal, opiate withdrawal, alcohol withdrawal acute behavioral disturbance, dementia, delirium, and restless legs syndrome in patients suffering from a viral or bacterial infection, said infection being due to a Rhinovirus, the Ebola virus, the measles virus or the Japanese encephalitis virus or to the bacteria *Pseudomonas aeruginosa, Staphylococcus aureus* or *Neisseriae gonorrhoeae.* All the above detailed embodiments can also be applied to any of these viral or bacterial - infected subjects, to prevent strong / severe forms of the infections and subsequent lethality to occur.

In this aspect, the following conventional doses of Diazepam can be administered to said patients:
- between 5-40 mg per day (2-10mg every 6-12h) for preventing anxiety or agitation orally,
- 2-10 mg every 6-8 hours, for preventing muscle spasms orally,
- 5-10 mg every 10 minutes, for treating epilepsy by intravenous or intramuscular use.

### Sedative use of Diazepam in COVID19 patients before medical procedures

In a third aspect, the present invention relates to the treatment of patients that have been infected by SARS-COV-2 (SARS-COV-2 positive subjects) or that have been exposed to SARS-COV-2 and that have to undergo a harmful or invasive medical procedure (e.g., operation or endoscopy). As a sedative agent, Diazepam will therefore advantageously induce sedation, anxiolysis or amnesia, before or after said medical procedure is performed, without worsening the outcome of the COVID disease, if the patient becomes infected. Also, it will prevent the worsening of the COVID 19 disease, if the patient is infected or likely to be infected.

In this aspect, the present invention is drawn to Diazepam for use for inducing pre-or post-operative sedation, anxiolysis, or amnesia, in patients suffering - or likely to suffer - from COVID 19 (SARS-COV-2 positive or exposed subjects), before medical chirurgical or invasive procedures occur. These medical invasive procedures usually occur in the Intensive Care Unit (ICU). Diazepam is therefore administered to these subjects before being transported to ICU, or in the ICU.

In these SARS-COV-2 positive or exposed subjects, Diazepam will prevent the worsening of the viral infection and will provide acceptable conditions for the invasive chirurgical procedure to take place (by placing the subject in an artificial sleep).

In this aspect, the following conventional sedative doses of Diazepam can be administered to said patients: -
- In the ICU: between 5-10 mg by intravenous administration one to two hours before the surgery begins, then 0.03-0.1mg/kg during the stay in ICU (e.g., between 30 minutes and 6 hours).
- Before endoscopy: between 5-10 mg by intramuscular administration, about 30 minutes before the endoscopy takes place,
- Before surgery: between 5 to 10 mg by intramuscular administration.

In this aspect, SARS-COV-2 positive or exposed subjects may have been previously treated with a BZRA or not. Preferably, these subjects are not currently treated with another BZRA of any kind.

Also, because of the similar molecular pathways involved in the entry of these pathogens in human cells (involving the ASM/ceramide system), the present inventors hereby recommend to use Diazepam as a sedative agent in patients that have to undergo a harmful or invasive medical procedure (e.g., operation or endoscopy), when said patients suffer from a viral or bacterial infection being due to a Rhinovirus, the Ebola virus, the measles virus or the Japanese encephalitis virus or to the bacteria *Pseudomonas aeruginosa, Staphylococcus aureus* or *Neisseriae gonorrhoeae.* All the above detailed embodiments can also be applied to any of these viral or bacterial - infected subjects, to prevent strong / severe forms of the infections and subsequent lethality to occur.

### Methods for prognosing the risk of mortality in COVID 19 patients

As explained in the examples below, the present inventors discovered that all BZRAs except Diazepam are associated with increased mortality when patients are hospitalized for COVID-19.

The present invention therefore also proposes an *in vitro* method for evaluating the risk of suffering from a severe COVID 19 disease, once infected with the SARS-COV-2 virus. This method is based on the information whether the patient is currently treated with a BZRA, and, if so, if said BZRA is Diazepam.

As explained thoroughly above, if a SARS-COV-2 positive subject is treated with a BZRA which is not Diazepam, then the prognosis of said subject is poor. Said subject is indeed more likely to suffer from a severe or critical form of COVID 19 than subjects that are not treated with a BZRA or subjects that are treated with Diazepam. Also, his / her vital prognosis is more engaged.

Also, the present invention gives a way to select patients that are likely to benefit from a BZRA treatment versus those who need to stop a BZRA treatment in order to avoid the worsening of the SARS-COV-2 infection. In these latter, said BZRA treatment should be taped off, and eventually stopped and Diazepam should be potentially administered instead.

Also, because of the similar molecular pathways involved in the entry of these pathogens in human cells (involving the ASM/ceramide system), the present inventors propose prognostic methods to assess the risk of mortality for patients suffering from a viral or bacterial infection being due to a Rhinovirus, the Ebola virus, the measles virus or the Japanese encephalitis virus or to the bacteria *Pseudomonas aeruginosa, Staphylococcus aureus* or *Neisseriae gonorrhoeae.* All the above detailed embodiments can also be applied to any of these viral or bacterial - infected subjects, if they are currently treated with a BZRA

### Pharmaceutical compositions

The agent of the invention (the BZRA compound, should it be Diazepam or another BZRA) is preferably contained in a pharmaceutical composition. This pharmaceutical composition, herein also called the "pharmaceutical composition of the invention", therefore comprises the BZRA agent of the invention and a pharmaceutically acceptable carrier.

A "pharmaceutically acceptable carrier" or "excipient" refers to a non-toxic solid, semi-solid or liquid filler, diluent, encapsulating material or formulation auxiliary of any type. In the pharmaceutical compositions of the present invention, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms. Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical compositions of the invention can contain any pharmaceutically acceptable salt of the agent of the invention (the BZRA compound, should it be Diazepam or another BZRA), or any functional derivatives, analogs, isomers, metabolites, solvate, clathrate, polymorphs or co-crystal thereof.

The term "pharmaceutically acceptable salt" is intended to mean, in the framework of the present invention, a salt of a compound which is pharmaceutically acceptable, as defined above, and which possesses the pharmacological activity of the corresponding compound.

The pharmaceutically acceptable salts comprise:
1) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic, and trifluoroacetic acid and the like, and
2) base addition salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

### FIGURE LEGENDS

**Figure 1** discloses the study cohort
**Figure 2** describes the Kaplan-Meier curves for mortality in the full sample crude analysis (N=14,381) (A), in the full sample analysis with inverse probability weighting (N=14,381) (B) and in the matched analytic sample using a 1:1 ratio (N=1,372) (C) of patients who had been hospitalized for COVID-19, according to benzodiazepine receptor agonist (BZRA) use.

The shaded areas represent pointwise 95% confidence intervals.

### EXAMPLES

Although the present invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

### I. MATERIAL AND METHODS

### 1.1. Setting and Cohort Assembly

A multicenter observational retrospective cohort study was conducted at AP-HP, which includes 39 hospitals. All adults were included, aged 18 years or over, who have been admitted to these medical centers from the beginning of the epidemic in France, i.e. January 24^{th}, until May 1^{st} 2020. COVID-19 was ascertained by a positive reverse-transcriptase-polymerase-chain-reaction (RT-PCR) test from analysis of nasopharyngeal or oropharyngeal swab specimens. This observational study using routinely collected data received approval from the Institutional Review Board of the AP-HP clinical data warehouse (decision CSE-20- 20_COVID19, IRB00011591).

### 1.2. Data sources

The data were from the AP-HP Health Data Warehouse ('Entrepôt de Données de Santé (EDS)'). This warehouse contains all available clinical data on all inpatient visits for COVID-19 to any Greater Paris University hospital. The data included patient demographic characteristics, vital signs, laboratory test and RT-PCR test results, medication administration data, medication lists during current and past hospitalizations in AP-HP hospitals, current diagnoses, discharge disposition, and death certificates.

### 1.3. Variables assessed

The following data were obtained for each patient at the time of the hospitalization through electronic health records: ^{18,19} sex, age, hospital, obesity, self-reported current smoking status, any medical condition associated with increased clinical severity related to COVID-19 or BZRA use, ²²⁻²⁵ any current psychiatric disorder, any medication prescribed according to compassionate use or as part of a clinical trial, ^{26,27} any psychotropic medication, including any antidepressant, ²⁸ mood stabilizer (i.e. lithium or antiepileptic medications with mood stabilizing effects), and antipsychotic medication, ^{29,30} respiratory depression, defined by a respiratory rate <12 breaths/min or a resting peripheral capillary oxygen saturation in ambient air < 90%, ²⁷ and other clinical markers of disease severity, defined as having temperature > 40°C or systolic blood pressure <100 mmHg or respiratory rate >24 breaths/min or plasma lactate levels higher than 2 mmol/L. ²⁷ For these two later variables, a third category for missing data was added.

### 1.4. Benzodiazepine receptor agonist (BZRA) use

Study baseline was defined as the date of hospital admission. BZRA use was defined as receiving these medications at baseline, i.e., within the first 48 hours of hospital admission, and before the end of the index hospitalization or death, to minimize potential confounding effects of late prescription of BZRAs, which can be used in palliative care. This delay was used because it was considered that, in a context of overwhelmed hospital units during the COVID-19 peak incidence, all patients may not have received or been prescribed their usual medication regimens the first day of their hospital admission, or this treatment may not have been recorded at this time.

### 1.5. Primary endpoint

The primary endpoint was the time from study baseline to death. Patients without an end-point event had their data censored on May 1^{st}, 2020.

### 1.6. Statistical analysis

The frequencies and means (± standard deviations (SD)) of all baseline characteristics described above were calculated in patients receiving or not receiving BZRAs and compared them using standardized mean differences (SMD).

To examine the association between BZRA use and the endpoint of death, we performed Cox proportional-hazards regression models. ³¹ To help account for the nonrandomized prescription of BZRAs and reduce the effects of confounding, the primary analysis used propensity score analysis with inverse probability weighting (IPW). ^{20,32} The individual propensities for receiving any BZRA were estimated using a multivariable logistic regression model that included patient characteristics (i.e., sex, age, hospital, obesity, current smoking status, any significant medical condition, and any current psychiatric disorder) and other medications, including any medication prescribed according to compassionate use or as part of a clinical trial and other psychotropic medications, including any antidepressant, mood stabilizer, or antipsychotic medication. In the inverse-probability-weighted analyses, the predicted probabilities from the propensity-score models were used to calculate the stabilized inverse-probability-weighting weights. ³² The association between BZRA use and mortality was then estimated using an IPW Cox regression model. In case of non-balanced covariates, an IPW multivariable Cox regression model adjusting for the non-balanced covariates was also performed. Kaplan-Meier curves were performed using the inverse-probability-weighting weights ^{33,34} and their pointwise 95% confidence intervals were estimated using the nonparametric bootstrap method. ^{34,35}

Five sensitivity analyses were conducted. First, a multivariable Cox regression model was performed, including as covariates the same variables as in the IPW analysis. Second, a univariate Cox regression model was used in a matched analytic sample using a 1:1 ratio, based on the same variables used for the IPW analysis and the multivariable Cox regression analysis. To reduce the effects of confounding, optimal matching was used in order to obtain the smallest average absolute distance across all clinical characteristics between exposed patients and non-exposed matched controls. ³⁶ Third, to examine a potential indication bias of prescription of BZRAs in ICUs as a possible treatment for palliative care or as an aid to oral intubation, the main analyses were reproduced after excluding all patients who had been hospitalized in ICUs. Fourth, it was examined whether these findings were similar in models imputing missing data using multiple imputation ³⁷ instead of excluding patients with any missing data as done in the main analyses. Finally, to examine the potential influence on the results of excluding patients who received BZRA after 48 hours from hospital admission, the main analysis was reproduced while including all participants who received any BZRA at any time from hospital admission until the end of the index hospitalization or death, and considering BZRA use as a time-dependent variable. ³¹ In that analysis, patients who received any BZRA after hospital admission entered the analysis risk-sets at the time of actual first initiation of the treatment. In all these analyses, weighted Cox regression models were used when the proportional hazards assumption was not met ³⁸.

Several additional exploratory analyses were performed. First, it was searched among patients who received a BZRA for a potential dose-dependent relationship by testing the association between daily dose of BZRA received at baseline (converted into diazepam-equivalent dose 39 and dichotomized at the mean value) and the endpoint, adjusted for the same covariates as used in the main analysis. Second, it was examined whether respiratory depression or other clinical markers of disease severity may at least partly explain this association by adjusting successively for these variables. Finally, it was examined the relationships between each BZRA and mortality using the same statistical approach as described for the main analysis.

For all associations, it was performed residual analyses to assess the fit of the data, check assumptions, including proportional hazards assumption using proportional hazards tests and diagnostics based on weighted residuals ^{31,40}, and examined the potential influence of outliers. To improve the quality of result reporting, the recommendations of The Strengthening the Reporting of Observational Studies in Epidemiology (STROBE) Initiative were followed. ³⁵ Because this main hypothesis focused on the association between BZRA use and mortality, statistical significance was fixed *a priori* at two-sided p-value <0.05. Only if a significant association were found in the main analysis, additional exploratory analyses were performed as described above. All analyses were conducted in R software version 2.4.3 (R Project for Statistical Computing).

### II. RESULTS

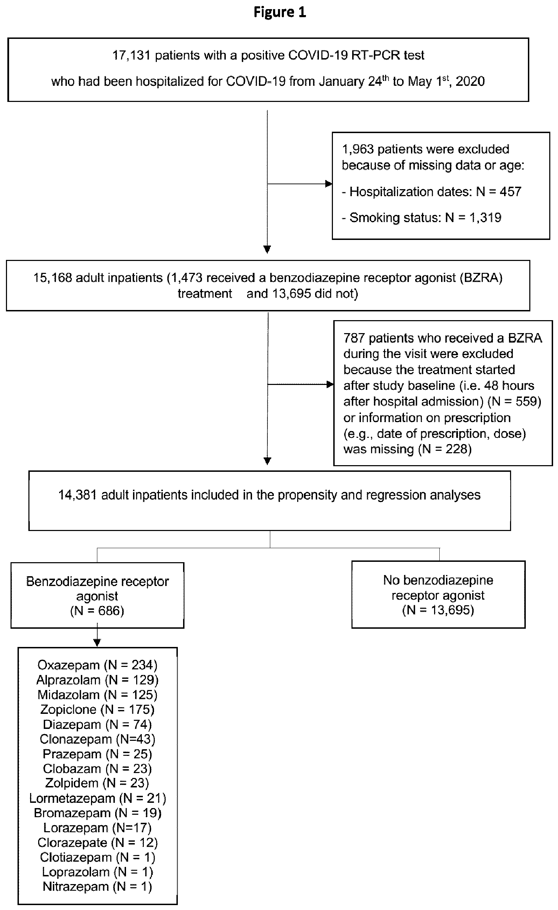

### 2.1. Characteristics of the cohort (figure 1)

Of the 17,131 patients with a positive COVID-19 RT-PCR test who had been hospitalized for COVID-19, 1,963 patients (11.5%) were excluded because of missing data or their young age (i.e. less than aged 18 years). In addition, of the 1,473 patients who received a BZRA at any time during the visit, 787 patients were excluded because they were prescribed the medication more than 48 hours after hospital admission. Of the remaining 14,381 adult patients, 686 (4.8%) received a BZRA in the first 48 hours of hospitalization at a mean diazepam-equivalent dose of 19.7 mg per day (SD=25.4, median=10.0, *lower quartile*=*5.0, higher quartile*=*22.9,* range=1.85-240.0 mg). Of these 686 patients, 41.1% (N=282) had a prescription of BZRA during a prior hospitalization at AP-HP in the past 6 months, and 36.4% (N=183) received at least two BZRA medications. The median delay from hospital admission to first prescription of BZRA was 0.94 days (SD=0.55).

RT-PCR test results were obtained after a median delay of 1.2 days (SD=12.7) from hospital admission date. This median delay was of 1.0 day (SD=11.6) in the exposed group and of 1.2 day (SD=12.8) in the non-exposed group.

Over a mean follow-up of 14.5 days (SD=18.1; median=7 days), 1,320 patients (9.2%) had an end-point event at the time of data cutoff on May 1, 2020. Among patients who received a BZRA, the mean follow-up was 12.8 days (SD=13.1, median=8 days), while it was of 14.5 days (SD=18.3, median=7 days) in those who did not.

All baseline characteristics were independently and significantly associated with the endpoint. A multivariable Cox regression model showed that age, sex, hospital, any medical condition, and any current psychiatric disorder were significantly and independently associated with the endpoint.

The distributions of patient characteristics according to BZRA use are shown in **Table 1.** In the full sample, BZRA use significantly differed according to all characteristics except for sex, and the direction of the associations indicated older age and greater medical severity of patients receiving a BZRA. After applying the propensity score weights, these differences were substantially reduced. In the matched analytic sample, there were no significant differences in any characteristic, except for a greater prevalence of men in the non-exposed group (**Table 1**).

**Table 1. Characteristics of patients with COVID-19 receiving or not receiving a benzodiazepine receptor agonist (BZRA) (N=14,381).**

| | Exposed to any BZRA (N=686) | Not exposed to any BZRA (N=13,695) | Non-exposed matched group (N=686) | Exposed to any BZRA vs. Not exposed | Exposed to any BZRA vs. Not exposed | Exposed to any BZRA vs. Non-exposed matched group |
|---|---|---|---|---|---|---|
| | | | | Crude analysis | Analysis weighted by inverse-probability-weighting weights | Matched analytic sample analysis |
| | N (%) | N (%) | N (%) | SMD | SMD | SMD |
| Age | | | | 0.893 | 0.174 | 0.029 |
| *18 to 50 years* | 70 | 5669 | 65 (9.4%) | | | |
| *51 to 70 years* | 192 | 4395 | 198 | | | |
| *More than 70 years* | 424 | 3631 | 423 | | | |
| Sex | | | | 0.047 | 0.095 | 0.108 |
| *Women* | 348 | 7270 | 311 | | | |
| *Men* | 338 (49.3%) | 6425 (46.9%) | 375 (54.7%) | | | |
| Hospital | | | | 0.387 | 0.050 | 0.063 |
| *AP-HP Centre* - *Paris University, Henri Mondor* | 206 (30.0%) | 6585 (48.1%) | 212 (30.9%) | | | |
| *AP-HP Nord and Hôpitaux Universitaires Paris* | 222 (32.4%) | 3685 (26.9%) | 227 (33.1%) | | | |
| *AP-HP Paris Saclay* | 122 | 1575 | 106 | | | |
| *AP-HP Sorbonne* | 136 | 1850 | 141 | | | |
| Obesity ^{α} | | | | 0.186 | 0.074 | 0.079 |
| *Yes* | 135 | 1758 | 114 | | | |
| *No* | 551 (80.3%) | 11937 (87.2%) | 572 (83.4%) | | | |
| Smoking ^{β} | | | | 0.263 | 0.074 | 0.065 |
| *Yes* | 112 | 1072 | 96 | | | |
| *No* | 574 (83.7%) | 12623 (92.2%) | 590 (86.0%) | | | |
| Any medical condition ^{β} | | | | 0.711 | 0.058 | 0.027 |
| *Yes* | 393 | 3336 | 402 | | | |
| *No* | 293 (42.7%) | 10359 (75.6%) | 284 (41.4%) | | | |
| Medication according to compassionate use or as part of a clinical trial ^{θ} | | | | 0.371 | 0.103 | 0.017 |
| *Yes* | 170 | 1483 | 165 | | | |
| *No* | 516 (75.2%) | 12212 (89.2%) | 521 (75.9%) | | | |
| Any current psychiatric | | | | 0.582 | 0.052 | 0.007 |
| *Yes* | 154 | 498 (3.6%) | 152 | | | |
| *No* | 532 (77.6%) | 13197 (96.4%) | 534 (77.8%) | | | |
| Any antidepressant | | | | 1.045 | 0.185 | 0.075 |
| *Yes* | 285 | 411 (3.0%) | 260 | | | |
| *No* | 401 (58.5%) | 13284 (97.0%) | 426 (62.1%) | | | |
| Any mood stabilizer | | | | 0.617 | 0.404 | 0.011 |
| *Yes* | 143 | 284 (2.1%) | 140 | | | |
| *No* | 543 (79.2%) | 13411 (97.9%) | 546 (79.6%) | | | |
| Any antipsychotic | | | | 0.714 | 0.305 | 0.014 |
| *Yes* | 163 | 198 (1.4%) | 159 | | | |
| *No* | 523 (76.2%) | 13497 (98.6%) | 527 (76.8%) | | | |

### 2.2. Study endpoint

The endpoint of death occurred in 186 patients (27.1%) who received a BZRA at baseline and 1,134 patients (8.3%) who did not. The crude, unadjusted analysis (hazard ratio (HR)=3.20; 95% CI=2.74-3.74; p<0.001), the primary analysis with inverse probability weighting (HR=1.61; 95% CI=1.31-1.98; p<0.001) and the multivariable IPW Cox regression adjusting for unbalanced covariates (HR=1.56; 95% CI=1.29-1.89; p<0.001) showed a significant association between BZRA use and increased mortality (**Figure 1**;

**Table 2**). A *post hoc* analysis indicated that there was 80% power in the crude analysis to detect a hazard ratio of at least 1.27.

**Table 2. Association between benzodiazepine receptor agonist (BZRA) use and mortality.**

| | **Number of events / Number of patients** | **Crude Cox regression analysis** | **Multivariable Cox regression analysis a** | **Analysis weighted by inverse-probability-weighting weights** | **Analysis weighted by inverse-probability-weighting weights adjusted for unbalanced covariates a** | **Number of events / Number of patients in the matched groups** | **Univariate Cox regression in a 1:1 ratio matched analytic sample** |
|---|---|---|---|---|---|---|---|
| | N (%) | HR (95% CI; p-value) | HR (95% CI; p-value) | HR (95% CI; p-value) | HR (95% CI; p-value) | N (%) | HR (95% CI; p-value) |
| No BZRA | 1,134 / 13,695 (8.3%) | Ref. | Ref. | Ref. | Ref. | 143/686 (20.8%) | Ref. |
| Any BZRA | 186/686 (27.1%) | 3.20 (2.74 - 3.74; <0.001*) | 1.94 (1.45 - 2.59; <0.001*) | 1.61 (1.31-1.98; <0.001*) | 1.56 (1.29 - 1.89; <0.001*) | 186/686 (27.1%) | 1.34 (1.08 - 1.67; 0.009*) |

| | | | | | | | |
|---|---|---|---|---|---|---|---|
| * Two-sided p-value is significant (p<0.05). ^{a} Adjusted for age, medication according to compassionate use or as part of a clinical trial, any mood stabilizer medication, and any antipsychotic medication. Abbreviations: HR, hazard ratio; CI, confidence interval. | | | | | | | |

In sensitivity analyses, the multivariable Cox regression model in the full sample also yielded a significant association (HR=1.94; 95% CI=1.45-2.59; p<0.001), as did the univariate Cox regression model in a matched analytic sample using a 1:1 ratio (HR=1.34; 95% CI=1.08-1.67; p=0.009) (**Table 2**). Similarly, the primary analysis using imputed data yielded significant results (**not shown**), as did that considering BZRA use as a time-dependent variable and including all patients who received a BZRA at any time during the visit (**not shown**). The exclusion from the analyses of the patients who had been admitted to ICUs did not alter the significance of the association (**not shown**).

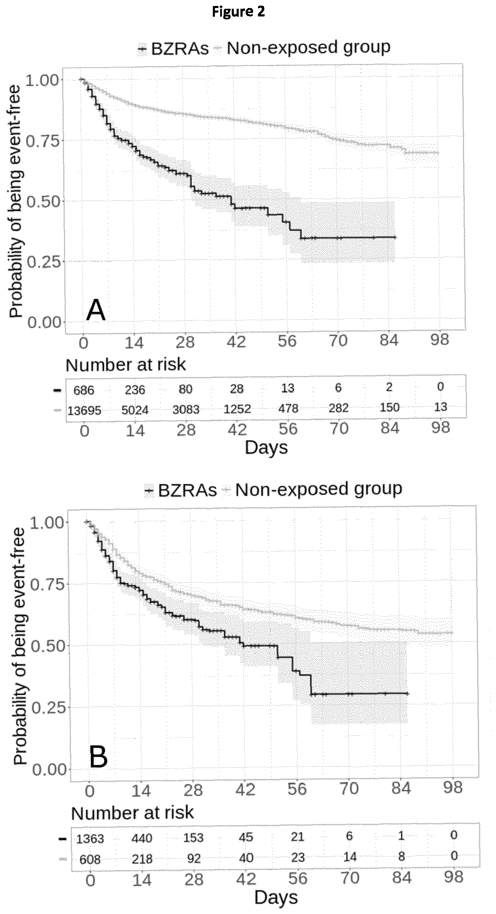

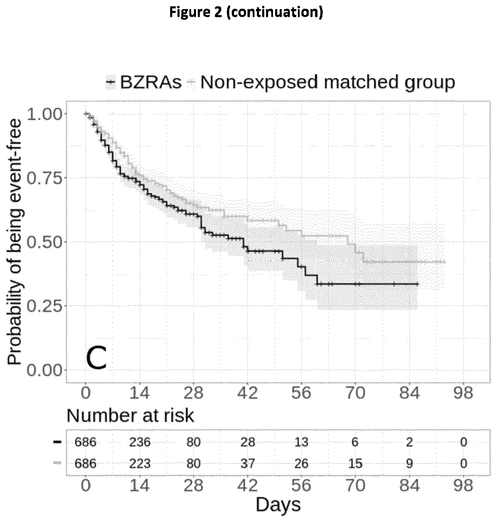

Additional analyses showed a significant dose-dependent relationship between baseline daily BZRA dose and mortality (HR=1.55; 95% CI=1.08-2.22; p=0.017). Additional adjustments for respiratory depression, any other clinical markers of disease severity, or both, resulted in still significant associations, which were of similar magnitude to that observed in the primary analysis (**not shown**). All individual BZRAs were significantly associated with increased mortality, except for diazepam (**Table 3**). Following adjustments, there were significant associations of any BZRA other than diazepam, midazolam, and any BZRA other than diazepam and midazolam with increased mortality, as compared to not receiving BZRAs (**Table 3**). Finally, compared with any other BZRA treatment, diazepam use was significantly associated with reduced mortality in the crude analysis (HR, 0.44; 95% CI, 0.23 to 0.86; p=0.017), in the primary analysis (HR, 0.31; 95% CI, 0.13 to 0.74; p=0.008), and in the sensitivity analyses (**Figure 2****; Table 3**).

**Table 3. Associations between individual benzodiazepine receptor agonists (BZRAs) and mortality.**

| | **Number of events / Number of patients** | **Crude Cox regression analysis** | **Multivariable Cox regression analysis ^{a}** | **Analysis weighted by inverse-probability-weighting weights** | **Analysis weighted by inverse-probability-weighting weights adjusted for unbalanced covariates** |
|---|---|---|---|---|---|
| | N (%) | HR (95%CI; p-value) | HR (95%CI; p-value) | HR (95%CI; p-value) | HR (95%CI; p-value) |
| No BZRA | 1,134 / 13,695 (8.3) | Ref. | Ref. | Ref. | Ref. |
| Diazepam | 9/74 (12.2) | 1.48 (0.77 - 2.86; 0.240) | 0.85 (0.40 - 1.82; 0.678) | 0.84 (0.29 - 2.46; 0.747) | 1.20 (0.56 - 2.60; 0.636) |
| Any BZRA other than diazepam | 177 / 612 (28.9) | 3.39 (2.90 - 3.98; <0.001*) | 2.02 (1.50 - 2.73; <0.001*) | 1.63 (1.33 - 2.01; <0.001*) | 1.68 (1.40 - 2.01; <0.001*) |
| Alprazolam | 32 / 129 (24.8) | 2.56 (1.80 - 3.63; <0.001*) | 1.19 (0.82 - 1.72; 0.366) | 1.22 (0.79 - 1.89; 0.359) | 1.23 (0.82 - 1.85; 0.987) |
| Clonazepam | 9/43 (20.9) | 2.19 (1.14 - 4.22; 0.019*) | 1.28 (0.65 - 2.50; 0.476) | 1.32 (0.65 - 2.69; 0.442) | 2.55 (0.98 - 6.65; 0.055) |
| Midazolam | 74 / 125 (59.2) | 8.34 (6.59 - 10.57; <0.001*) | 3.72 (2.90 - 4.78; <0.001*) | 3.02 (2.29 - 3.99; <0.001*) | 3.06 (2.35 - 4.01; <0.001*) |
| Oxazepam | 57/234 (24.4) | 3.05 (1.97 - 4.72; <0.001*) | 1.59 (0.96 - 2.61; 0.070) | 1.64 (1.16 - 2.31; 0.005*) | 1.44 (1.06 - 1.97; 0.022*) |
| Z-drugs (zopiclone or zolpidem) | 42/195 (21.5) | 3.08 (1.89 - 5.03; <0.001*) | 1.79 (0.98 - 3.24; 0.057) | 1.98 (1.22 - 3.22; 0.006*) | 1.39 (0.96 - 2.00; 0.081) |
| Other BZRAs | 23 / 129 (17.8) | 1.95 (1.29 - 2.95; 0.002*) | 1.12 (0.66 - 1.90; 0.688) | 1.72 (0.92 - 3.20; 0.089) | 1.27 (0.63 - 2.54; 0.500) |
| Any benzodiazepine receptor agonist other than diazepam or midazolam | 104/494 (21.1) | 2.76 (1.99 - 3.83; <0.001*) | 1.60 (1.09 - 2.35; 0.018*) | 2.13 (1.52 - 2.98; <0.001*) | 1.71 (1.22 - 2.38; 0.002*) |

| | | | | | |
|---|---|---|---|---|---|
| * Two-sided p-value is significant (p<0.05). ^{a} Adjusted for sex, age, hospital, obesity, current smoking status, any significant medical, any psychiatric condition, any medication prescribed according to compassionate use or as part of a clinical trial, and other psychotropic medications including any antidepressant, any mood stabilizer, and any antipsychotic medication. Abbreviations: HR, hazard ratio; CI, confidence interval. Only individual BZRAs associated with more than 5 end-point events are presented in the table. | | | | | |

### III. DISCUSSION

In the present multicenter retrospective observational study involving a large sample of patients hospitalized for COVID-19, benzodiazepine receptor agonist use was significantly and substantially associated with increased mortality, independent of patient characteristics and other medications, and with a significant dose-dependent relationship. This association remained significant in multiple sensitivity analyses. Secondary exploratory analyses suggested that most BZRAs were associated with this risk, with the possible exception of diazepam, for which mortality was significantly lower than in patients receiving any other BZRA and not significantly different than in patients not receiving BZRAs.

Thus, BZRA use was significantly associated with increased mortality among patients hospitalized for COVID-19. This association might be explained by several mechanisms. First, although the risk of respiratory depression associated with benzodiazepines in the general population is debated and was not found to play a substantial role in our study, it might be relevant among elderly patients with COVID-19 and in those with pre-existing comorbidities, such as chronic obstructive pulmonary disease. ⁴¹ Second, benzodiazepines might be associated with an increased risk of secondary infections, particularly pneumonia, in patients with COVID-19. ⁴² Finally, in patients with COVID-19 and known risk factors for delirium (e.g., old age, dementia, multiple comorbidities), BZRA use may favor the occurrence of this condition, ⁴¹ which is associated with unfavorable COVID-19 disease prognosis. ⁴³ These results suggest a need to carefully reevaluate the indication of BZRAs and to decrease dose or taper these medications when possible in patients with COVID-19, in line with professional guidelines recommendation for most patients on long-term benzodiazepines ⁵.

Secondary exploratory analyses indicate that most BZRAs may be associated with increased mortality among patients hospitalized for COVID-19, except diazepam. Interestingly, this result could be in line with preclinical findings. A prior study indicated a role of acid sphingomyelinase/ceramide system in SARS-CoV-2 infections ⁴⁴ and that the formation of ceramide-enriched membrane platforms may mediate the entry of the virus into epithelial cells. Although most BZRAs do not influence cellular ceramide levels, diazepam has been shown to interact with the acyl coenzyme A binding protein, also known as 'diazepam binding inhibitor', a protein that contributes to ceramide synthesis. ^{45,21} This interaction may result in a reduced activity of the ceramide synthase pathway and, finally, reduced cellular ceramide concentration, which might possibly explain reduced adverse effects of diazepam on COVID-19 compared to other BZRAs. However, given the exploratory nature of these analyses, the observational design of the study and the long half-life of diazepam, this result should be interpreted with caution and other studies are required to confirm this finding and this potential underlying mechanism.

Our study has several limitations. First, there are two major inherent potential biases in observational studies: unmeasured confounding and confounding by indication. We tried to minimize the effects of confounding in several different ways. First, we used an analysis with inverse probability weighting to minimize the effects of confounding by indication ^{20,32}. Second, we performed multiple sensitivity analyses, which showed similar results. Finally, although some amount of unmeasured confounding may remain, our analyses adjusted for numerous potential confounders. Other limitations include missing data for some baseline characteristic variables (i.e., 11.5%), which might be explained by the overwhelming of all hospital units during the COVID-19 peak incidence, and different results might have been observed during a lower COVID-19 incidence period. However, imputation of missing data did not alter the significance of our results. Second, inflation of type I error might have occurred in secondary analyses due to multiple testing. Third, our study cannot establish a causal relationship between BZRA use and increased mortality.

In this multicenter observational retrospective study, benzodiazepine receptor agonist use was significantly and substantially associated with increased mortality among adult patients hospitalized for COVID-19, with a significant dose-dependent relationship, suggesting a potential benefit of decreasing dose or tapering off these medications when possible in these patients. Additional studies are needed to determine the exact mechanisms underlying this association.

### References

1. Hoertel N, Blachier M, Blanco C, et al. A stochastic agent-based model of the SARS-CoV-2 epidemic in France. Nat Med. 2020;26(9):1417-1421. doi:https://doi.org/10.1038/s41591-020-1001-6
2. Hoertel N, Blachier M, Blanco C, et al. Facing the COVID-19 epidemic in NYC: a stochastic agent-based model of various intervention strategies. medRxiv. Published online 2020. doi:10.1101/2020.04.23.20076885
3. Chevance A, Gourion D, Hoertel N, et al. Ensuring mental health care during the SARS-CoV-2 epidemic in France: A narrative review. L'Encephale. 2020;46(3S): 193-201. doi: 10.1016/j.encep.2020.03.001
4. Mallon L, Broman J-E, Hetta J. Is usage of hypnotics associated with mortality? Sleep Med. 2009;10(3):279-286. doi:10.1016/j.sleep.2008.12.004
5. Hayhoe B, Lee-Davey J. Tackling benzodiazepine misuse: the time to take decisive action has come. BMJ. Published online July 27, 2018.
6. Belleville G. Mortality Hazard Associated with Anxiolytic and Hypnotic Drug Use in the National Population Health Survey. Can J Psychiatry. 2010;55(9):558-567. doi:10.1177/0706743710055009047.
7. Parsaik AK, Mascarenhas SS, Khosh-Chashm D, et al. Mortality associated with anxiolytic and hypnotic drugs-A systematic review and meta-analysis. Aust N Z J Psychiatry. 2016;50(6):520-533. doi:10.1177/0004867415616695
8. Weich S, Pearce HL, Croft P, et al. Effect of anxiolytic and hypnotic drug prescriptions on mortality hazards: retrospective cohort study. BMJ. 2014;348. doi:10.1136/bmj.g1996
9. Palmaro A, Dupouy J, Lapeyre-Mestre M. Benzodiazepines and risk of death: Results from two large cohort studies in France and UK. Eur Neuropsychopharmacol J Eur Coll Neuropsychopharmacol. 2015;25(10):1566-1577. doi:10.1016/j.euroneuro.2015.07.006
10. Patorno E, Glynn RJ, Levin R, Lee MP, Huybrechts KF. Benzodiazepines and risk of all cause mortality in adults: cohort study. BMJ. Published online July 6, 2017:j2941. doi:10.1136/bmj.j2941
11. Kojima M, Wakai K, Kawamura T, et al. Sleep patterns and total mortality: a 12-year follow-up study in Japan. J Epidemiol. 2000;10(2):87-93. doi:10.2188/jea.10.87
12. Phillips B, Mannino DM. Does insomnia kill? Sleep. 2005;28(8):965-971. doi:10.1093/sleep/28.8.965
13. Rumble R, Morgan K. Hypnotics, sleep, and mortality in elderly people. J Am Geriatr Soc. 1992;40(8):787-791. doi:10.1111/j.1532-5415.1992.tb01850.x
14. Joya FL, Kripke DF, Loving RT, Dawson A, Kline LE. Meta-analyses of hypnotics and infections: eszopiclone, ramelteon, zaleplon, and zolpidem. J Clin Sleep Med JCSM Off Publ Am Acad Sleep Med. 2009;5(4):377-383.
15. Obiora E, Hubbard R, Sanders RD, Myles PR. The impact of benzodiazepines on occurrence of pneumonia and mortality from pneumonia: a nested case-control and survival analysis in a population-based cohort. Thorax. 2013;68(2):163-170. doi:10.1136/thoraxjnl-2012-202374
16. Nakafero G, Sanders RD, Nguyen-Van-Tam JS, Myles PR. Association between benzodiazepine use and exacerbations and mortality in patients with asthma: a matched case-control and survival analysis using the United Kingdom Clinical Practice Research Datalink. Pharmacoepidemiol Drug Saf. 2015;24(8):793-802. doi:10.1002/pds.3799
17. Roth T. Hypnotic use for insomnia management in chronic obstructive pulmonary disease. Sleep Med. 2009;10(1):19-25. doi:10.1016/j.sleep.2008.06.005
18. Devlin J, Chang M-W, Lee K, Toutanova K. Bert: Pre-training of deep bidirectional transformers for language understanding. arXiv. Published online 2018. doi:1810.04805
19. Jouffroy J, Feldman SF, Lerner I, Rance B, Neuraz A, Burgun A. MedExt: combining expert knowledge and deep learning for medication extraction from French clinical texts. Published online January 23, 2020.
20. Robins JM, Hernán MÁ, Brumback B. Marginal Structural Models and Causal Inference in Epidemiology. Epidemiology. 2000;11(5):550-560.
21. Guidotti A, Forchetti CM, Corda MG, Konkel D, Bennett CD, Costa E. Isolation, characterization, and purification to homogeneity of an endogenous polypeptide with agonistic action on benzodiazepine receptors. ProcNatl Acad Sci. 1983;80(11):3531-3535.
22. Williamson E, Walker AJ, Bhaskaran KJ, et al. OpenSAFELY: factors associated with COVID-19-related hospital death in the linked electronic health records of 17 million adult NHS patients. MedRxiv. Published online 2020. doi:https://doi.org/10.1101/2020.05.06.20092999
23. Gordon DE, Jang GM, Bouhaddou M, et al. A SARS-CoV-2 protein interaction map reveals targets for drug repurposing. Nature. 2020;583(7816):459-468. doi:https://doi.org/10.1038/s41586-020-2286-9
24. Ruan Q, Yang K, Wang W, Jiang L, Song J. Clinical predictors of mortality due to COVID-19 based on an analysis of data of 150 patients from Wuhan, China. Intensive Care Med. 2020;46(5):846-848.
25. Hur K, Price CP, Gray EL, et al. Factors Associated With Intubation and Prolonged Intubation in Hospitalized Patients With COVID-19. Otolaryngol Neck Surg. Published online 2020.
26. Hoertel N, Sánchez M, Vernet R, et al. Dexamethasone use and Mortality in Hospitalized Patients with Coronavirus Disease 2019: a Multicenter Retrospective Observational Study. medRxiv. Published online 2020. doi:https://doi.org/10.1101/2020.10.23.20218172
27. Haut Conseil de la Santé Publique. Statement on the Management at Home or in a Care Facility of Suspected or Confirmed Covid-19 Patients.; 2020. https://www.hcsp.fr
28. Hoertel N, Sanchez-Rico M, Vernet R, et al. Association between SSRI antidepressant use and reduced risk of intubation or death in hospitalized patients with coronavirus disease 2019: A multicenter retrospective observational study. medRxiv. Published online 2020. doi:https://doi.org/10.1101/2020.07.09.20143339
29. Hoertel N, Sanchez Rico M, Vernet R, et al. Observational Study of Chlorpromazine in Hospitalized Patients with Covid-19. medRxiv. Published online 2020. doi:10.1101/2020.07.15.20154310
30. Hoertel N, Rico MS, Vernet R, et al. Observational Study of Haloperidol in Hospitalized Patients with Covid-19. medRxiv. Published online 2020. doi:https://doi.org/10.1101/2020.07.15.20150490
31. Terry M. Therneau, Patricia M. Grambsch. Modeling Survival Data: Extending the Cox Model. Springer; 2000.
32. Geleris J, Sun Y, Platt J, et al. Observational Study of Hydroxychloroquine in Hospitalized Patients with Covid-19. N Engl J Med. 2020;382(25):2411-2418. doi:10.1056/NEJMoa2012410
33. Efron B. Nonparametric standard errors and confidence intervals. Can J Stat. 1981;9(2):139-158.
34. Kassambara A, Kosinski M, Biecek P. Survminer: Drawing Survival Curves Using "Ggplot2."; 2020. https://CRAN.R-project.org/package=survminer
35. Von Elm E, Altman DG, Egger M, Pocock SJ, Gøtzsche PC, Vandenbroucke JP. The Strengthening the Reporting of Observational Studies in Epidemiology (STROBE) statement: guidelines for reporting observational studies. Ann Intern Med. 2007;147(8):573-577.
36. Hansen BB, Klopfer SO. Optimal full matching and related designs via network flows. J Comput Graph Stat. 2006;15(3):609-627.
37. Stekhoven DJ, Buehlmann P. MissForest - non-parametric missing value imputation for mixed-type data. Bioinformatics. 2012;28(1):112-118.
38. Dunkler D, Ploner M, Schemper M, Heinze G. Weighted Cox Regression Using the R Package coxphw. J Stat Softw. 2018;84(2):1-26. doi:10.18637/jss.v084.i02
39. Ashton CH. Benzodiazepines: How they work and how to withdraw. Ashton Man Aug. Published online 2002.
40. Grambsch PM, Therneau TM. Proportional hazards tests and diagnostics based on weighted residuals. Biometrika. 1994;81(3):515-526.
41. Ostuzzi G, Papola D, Gastaldon C, et al. Safety of psychotropic medications in people with COVID-19: evidence review and practical recommendations. BMC Med. 2020;18(1):1-14.
42. Sun G, Zhang L, Zhang L, Wu Z, Hu D. Benzodiazepines or related drugs and risk of pneumonia: A systematic review and meta-analysis. Int J Geriatr Psychiatry. 2019;34(4):513-521.
43. Chen T, Wu D, Chen H, et al. Clinical characteristics of 113 deceased patients with coronavirus disease 2019: retrospective study. BMJ. 2020;368. doi:10.1136/bmj.m1091
44. Carpinteiro A, Edwards MJ, Hoffmann M, et al. Pharmacological inhibition of acid sphingomyelinase prevents uptake of SARS-CoV-2 by epithelial cells. Cell Rep Med. Published online 2020:100142.
45. Ferreira NS, Engelsby H, Neess D, et al. Regulation of very-long acyl chain ceramide synthesis by acyl-CoA-binding protein. J Biol Chem. 2017;292(18):7588-7597.
46. Grassmé H, Jekle A, Riehle A et al. CD95 signaling via ceramide-rich membrane rafts. J Biol Chem 2001;276:20589-20596.
47. Grassmé H, Jendrossek V, Riehle A et al. Host defense against Pseudomonas aeruginosa requires ceramide-rich membrane rafts. Nat Med 2003;9:322-330.
48. Grassmé H, Riehle A, Wilker B, Gulbins E. Rhinoviruses infect human epithelial cells via ceramide-enriched membrane platforms. J Biol Chem 2005;280:26256-26262.
49. Miller ME, Adhikary S, Kolokoltsov AA, Davey RA. Ebolavirus requires acid sphingomyelinase activity and plasma membrane sphingomyelin for infection. J Virol 2012;86:7473-7483.
50. Avota E, Gulbins E, Schneider-Schaulies S. DC-SIGN mediated sphingomyelinase-activation and ceramide generation is essential for enhancement of viral uptake in dendritic cells. PLOS Pathog 2011;7:e1001290.
51. Tani H, Shiokawa M, Kaname Y et al. Involvement of ceramide in the propagation of Japanese encephalitis virus. J Virol 2010;84:2798-2807.
52. Esen M, Schreiner B, Jendrossek V et al. Mechanisms of Staphylococcus aureus induced apoptosis of human endothelial cells. Apoptosis 2001;6:431-439.
53. Grassmé H, Gulbins E, Brenner B et al. Acidic sphingomyelinase mediates entry of N. gonorrhoeae into nonphagocytic cells. Cell 1997;91:605-615.
54. Hauck CR, Grassmé H, Bock J et al. Acid sphingomyelinase is involved in CEACAM receptor-mediated phagocytosis of Neisseria gonorrhoeae. FEBS Lett 2000;478:260-266.
55. Shivanna V, Kim Y, Chang KO. Ceramide formation mediated by acid sphingomyelinase facilitates endosomal escape of caliciviruses. Virology 2015;483:218-228.

## Claims

1. Diazepam for use in preventing severe forms of COVID19 disease to occur in SARS-COV-2 positive subjects or SARS-COV-2 exposed subjects that are treated with a Benzodiazepine Receptor Agonist (BZRA) other than Diazepam.

2. Diazepam for use according to claim 1, for preventing and/or treating agitation, anxiety, panic, muscle spasms, benzodiazepine withdrawal, opiate withdrawal, alcohol withdrawal, trouble sleeping, dementia, acute behavioral disturbance, delirium and restless legs syndrome, in said SARS-COV-2 positive subjects or SARS-COV-2 exposed subjects.

3. Diazepam for use according to claim 1, for preventing epileptic seizure in and/or treating epilepsy in SARS-COV-2 positive or exposed subjects.

4. Diazepam for use according to claim 1, for inducing pre-or post-operative sedation, anxiolysis, or amnesia in SARS-COV-2 positive or exposed subjects.

5. Diazepam for use in inducing pre-or post-operative sedation, anxiolysis, or amnesia, in SARS-COV-2 positive subjects or in SARS-COV-2 exposed subjects, before a medical invasive procedure.

6. Diazepam for use according to claim 5, wherein Diazepam further prevents severe forms of the COVID19 disease to develop.

7. Diazepam for use according to claim 5 or 6, wherein said medical invasive procedure is a chirurgical operation or an endoscopy.

8. Diazepam for use according to any of claim 1 - 7, wherein said SARS-COV-2 positive subjects have been diagnosed by molecular biological detection and/or viral titration of respiratory specimens, or by assaying blood for antibodies specific for said betacoronavirus.

9. Diazepam for use according to any of claim 1 - 7, wherein said SARS-COV-2 exposed subjects have been in unprotected close contact with a SARS-COV-2 positive subject.

10. Diazepam for use according to any of claim 1 - 7, wherein said SARS-COV-2 exposed subjects have been in contact with a SARS-COV-2 positive subject without effective protection at less than two meters, preferably less than one meter, whatever the duration of the contact.

11. Diazepam for use according to claim 10, wherein said effective protection is a surgical mask, a FFP2 mask, a cloth mask or an hygiaphone.

12. A Benzodiazepine Receptor Agonist (BZRA) for use in the prevention and/or treatment of anxiety, panic disorders, insomnia, seizures, benzodiazepine withdrawal, opiate withdrawal, alcohol withdrawal, sedation, depression, delirium, acute behavioral disturbance, dementia and/or spasticity, wherein said BZRA is used only in subjects that have been negatively tested for SARS-COV-2 infection by molecular biological detection and/or viral titration of respiratory specimens, or by assaying blood for antibodies specific for said betacoronavirus.

13. BZRA for use according to claim 12, wherein said subjects have been efficiently immunized against the SARS-COV-2 virus by vaccinal immunization.

14. An *in vitro* method for evaluating the risk of a SARS-COV-2 positive subject to suffer from a severe form of the COVID 19 disease, said method comprising determining whether the subject is currently treated with a Benzodiazepine Receptor Agonist (BZRA).

15. The method of claim 14, wherein, if a SARS-COV-2 positive subject is treated with a BZRA which is not Diazepam, then the prognosis of said subject is poor.
